# EUROPEAN PATENT SPECIFICATION

(11) **EP 0 781 760 B1**
(45) Date of publication and mention of the grant of the patent: **04.09.2002**
(21) Application number: 96309106.1
(22) Date of filing: 13.12.1996
(51) Int. Cl.: C07C 68/06, C07C 69/96

(54) **Continuous manufacturing method for aromatic carbonates**
Verfahren zur kontinuierlichen Herstellung von aromatischen Carbonaten
Méthode de fabrication continue de carbonates aromatiques

(30) Priority: 27.12.1995 JP 34151095
(43) Date of publication of application: 02.07.1997
(73) Proprietor: GENERAL ELECTRIC COMPANY, Schenectady, NY 12345 (US)
(72) Inventor: Inoki, Satoshi, Otake City, Hiroshima Prefecture (JP); Tanaka, Michio, Otake City, Hiroshima Prefecture (JP); Motoyama, Yoshio, Saeki gun, Hiroshima Prefecture (JP); Uno, Kazutoyo, Chiba City, Chiba Prefecture (JP)
(74) Representative: Szary, Anne Catherine, Dr.

(56) References cited:
- EP-A- 0 461 274
- EP-A- 0 582 931
- WO-A-92/18458

## Description

The present invention concerns continuous manufacturing methods for aromatic carbonates; more specifically, it concerns a manufacturing method by which aromatic carbonates can be continuously and efficiently manufactured from dialkyl carbonate and an aromatic hydroxy compound.

Diphenyl carbonate (DPC) is an industrially useful compound that is used as a raw material in the manufacture of polycarbonate and the ability to manufacture diphenyl carbonate productively is an industrially useful skill.

It is known in the prior art to obtain diphenyl carbonate by reacting dialkyl carbonate and an aromatic hydroxy compound.

For example, methyl phenyl carbonate, diphenyl carbonate, or mixtures of them can be obtained as follows by reacting dimethyl carbonate and phenol.

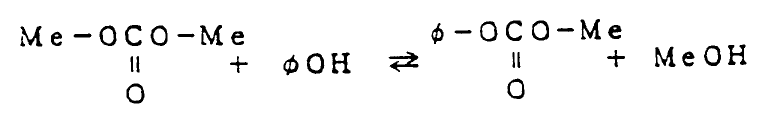

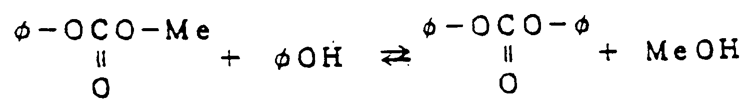

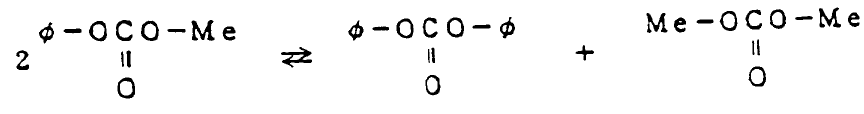

However, the problem with such reactions is that they are all equilibrium reactions that tend to lead back to the original systems and they have slow reaction rates.

In an attempt to solve these problems, various catalysts for raising the reaction rate have been proposed.

Japanese Unexamined Patent Application Disclosure Kokai No. Hei 3-291257 discloses a method for continuously manufacturing aromatic carbonates in which alcohols and other by-products are continuously distilled off with a multistage distillation column to promote the productivity of the reaction, as the reaction product is continuously removed.

It is also known to use reactors equipped with distilling columns in an attempt to distill off alcoholic by-products of the reaction (e.g., methyl alcohol) from the raw materials, product and solvent and promote a more productive reaction.

In continuous methods for manufacturing aromatic carbonates such as those mentioned hereinabove, unreacted raw materials (including dialkyl carbonate by-products of the reaction), catalyst and solvent are recovered from the reactor along with the reaction products and alcoholic by-products. These continuous methods are efficient because these materials, especially the unreacted raw materials, are returned to the reaction system.

However, in their studies of such methods for continuously manufacturing aromatic carbonates, the present inventors discovered that the efficiency at which aromatic carbonates are produced in a continuous operation diminishes as recovered unreacted raw materials are returned to the reaction system. As they studied the cause of this, the present inventors discovered that the reaction of dialkyl carbonate and an aromatic hydroxy compound produces alkyl aromatic ether by-products such as anisole, albeit at low rates of selectivity, and that in a continuous operation these alkyl aromatic ethers accumulate in the reactor when unreacted raw materials are returned to the reaction system, diminishing the effective capacity of the reactor. Armed with this information, they also discovered that the efficiency of continuous manufacturing methods for aromatic carbonates can be improved by separating and removing alkyl aromatic ethers from unreacted raw materials removed from the reactor before recycling the unreacted raw materials back to the reaction system. This discovery completed the invention.

The object of the invention is to provide a method for continuously and efficiently manufacturing aromatic carbonates by reacting dialkyl carbonate and an aromatic hydroxy compound that is based on the research described hereinabove.

The continuous manufacturing method for aromatic carbonates of the invention is characterized by carrying out at least one of the refinement and recycling processes hereinbelow in a method for continuously manufacturing aromatic carbonates in a reactor by reacting dialkyl carbonate and an aromatic hydroxy compound in the presence of a catalyst, while continuously removing aromatic carbonate reaction product, alcoholic by-products, dialkyl carbonate, and aromatic hydroxy compound and while recovering and recycling to the reaction system dialkyl carbonate and aromatic hydroxy compound:
[I] a refinement and recycling process in which alcohols and alkyl aromatic ethers are separated and removed from the reaction mixture from the top of the reactor, this reaction mixture containing alcoholic by-products, dialkyl carbonates, and alkyl aromatic ether by-products formed in the reactor, and the dialkyl carbonate thus obtained is recycled to the reaction system
   and
[II] a refinement and recycling process in which the reaction mixture removed from the bottom of the reactor, this reaction mixture containing aromatic carbonates, dialkyl carbonates, aromatic hydroxy compound, and alkyl aromatic ether by-products formed in the reactor, is distilled, aromatic carbonate is removed from the bottom of the distilling column, alkyl aromatic ethers are separated and removed from the reaction mixture obtained at the top of the distilling column, and the dialkyl carbonate and/or aromatic hydroxy compound thus obtained are returned to the reaction system.

In the present invention, the aromatic carbonate is an alkyl aryl carbonate, a diaryl carbonate, or mixtures of them.

The preferred dialkyl carbonate is dimethyl carbonate or diethyl carbonate.

The preferred aromatic hydroxy compound is phenol, *m*- and /or *p*-cresol.

Typical examples of an alkyl aromatic ether are anisole, phenetole, methyl tolyl ether and ethyl tolyl ether.

When two reactors are used in the method for continuously manufacturing aromatic carbonates of the invention, it is preferred that

in the first reactor, alkyl aryl carbonates are produced as the aromatic carbonates by reacting dialkyl carbonate and an aromatic hydroxy compound in the presence of a catalyst
and then in the second reactor, diaryl carbonates are produced as the aromatic carbonate by either reacting the alkyl aryl carbonate obtained in the first reactor or by reacting the alkyl aryl carbonate and an aromatic hydroxy compound.

First to be described are the dialkyl carbonate, aromatic hydroxy compound, and catalyst used to manufacture aromatic carbonates in the present invention.

Dialkyl carbonates described by general formula (i) hereinbelow are used in the invention. 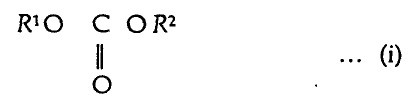 where *R*¹ and *R*² are alkyl groups, alkenyl groups, alicyclic groups, or aralkyl groups, *R*¹ and *R*² may be the same or different, and they may form a ring.

Specific examples of *R*¹ and *R*² include alkyl groups such as methyl groups, ethyl groups, propyl groups, butyl groups, pentyl groups, hexyl groups, heptyl groups, octyl groups, nonyl groups, and decyl groups;
alkenyl groups, allyl groups, and butenyl groups;
alicyclic groups such as cyclopropyl groups, cyclobutyl groups, cyclopentyl groups, cyclohexyl groups, and cycloheptyl groups;
alkyl groups containing alicyclic groups such as cyclohexylmethyl groups; and
aralkyl groups such as benzyl groups, phenethyl groups, phenylpropyl groups, phenylbutyl groups, and methylbenzyl groups.

These groups may be substituted with cyano groups, and halogen, and they may contain unsaturated bonds.

Examples of dialkyl carbonate described by formula (i) include dimethyl carbonate, diethyl carbonate, dipropyl carbonate, diallyl carbonate, dibutenyl carbonate, dibutyl carbonate, dipentyl carbonate, dihexyl carbonate, diheptyl carbonate, dioctyl carbonate, dinonyl carbonate, didecyl carbonate, methyl ethyl carbonate, methyl propyl carbonate, methyl butyl carbonate, ethyl propyl carbonate, ethyl butyl carbonate, ethylene carbonate, propylene carbonate, di(methoxymethyl) carbonate, di(methoxyethyl) carbonate, di(chloroethyl) carbonate, di(cyanoethyl) carbonate,
dicyclopentyl carbonate, dicyclohexyl carbonate, dicycloheptyl carbonate,
dibenzyl carbonate, diphenethyl carbonate, di(phenylpropyl) carbonate, di(phenylbutyl) carbonate, di(chlorobenzyl) carbonate, and di(methoxybenzyl) carbonate.

These dialkyl carbonates may be used in combinations of two or more.

Preferred among these is dialkyl carbonate where each *R*¹ and *R*² is an alkyl group with no more than four carbon atoms, with dimethyl carbonate and diethyl carbonate more preferred and dimethyl carbonate especially preferred.

The aromatic hydroxy compound used to manufacture aromatic carbonates is described by general formula (ii) hereinbelow.

Ar¹OH (ii)

Ar¹ is a monovalent aromatic group which may have a substituent.

Examples of such aromatic monohydroxy compounds include phenol and alkylphenols such as cresol, xylenol, trimethylphenol, tetramethylphenol, ethylphenol, propylphenol, butylphenol, diethylphenol, methylethylphenol, methylpropylphenol, dipropylphenol, methylbutylphenol, pentylphenol, hexylphenol, and cyclohexylphenol;
alkoxyphenols such as methoxyphenol and ethoxyphenol;
naphthols and substituted naphthols;
and substituted phenols described by the formula 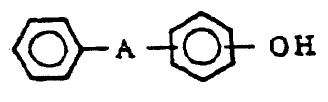 where *A* is -O-, -S-, -CO-, SO₂-; 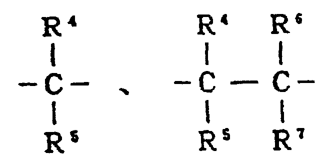 a (substituted) alkylene such as - a divalent group such as cycloalkylene, for example 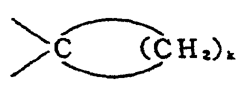 or simply a bond.
In these formulas, each *R*⁴, *R*⁵, *R*⁶, and *R*⁷ is a hydrogen atom, cycloalkyl group, aryl group,or aralkyl group, which may be substituted with a halogen atom or alkoxy group. *k* is an integer ranging in value from 3 to 11, and the hydrogen atoms may be replaced by aryl groups, halogen atoms, or the like.

Furthermore, alicyclic groups may be substituted with substituents such as ester groups, hydroxyl groups, nitro groups, halogen, and cyano groups.

Heteroaromatic hydroxy compounds such as hydroxypyridine, hydroxycoumarin, and hydroxyquinoline can also be cited as examples of the aromatic hydroxy compound.

Aromatic dihydroxy compounds can also be used, examples including hydroquinone, resorcinol, catechol, dihydroxynaphthalene, dihydroxyanthracene, and alkylated forms of them. Aromatic dihydroxy compounds described by the following formula can also be used. 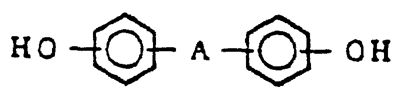 where *A* is identical to *A* hereinabove, and the alicyclic may be substituted with a substituent such as an ester group, hydroxyl group, nitro group, halogen, or cyano group.

In the present invention, aromatic monohydroxy compounds in which Ar¹ in the above-mentioned formula (ii) is an aromatic group with six to 10 carbon atoms are preferred, with phenol, *m*-cresol, and/or *p*-cresol being more preferred, and phenol being especially preferred. Aromatic hydroxy compounds may be used in combinations of two or more.

In the present invention, it is preferred that the aromatic hydroxy compound, examples of which are cited hereinabove, be fed to the reaction system in amounts such that the dialkyl carbonate/aromatic hydroxy compound molar ratio ranges from 10 to 1, more preferably 5 to 1.5.

Alkyl aryl carbonates and diallyl carbonates are produced from the above-described dialkyl carbonates and aromatic hydroxy compounds by reactions such as the following. The following examples are reactions using dialkyl carbonates in which *R*¹ and *R*² in formula (i) are the same.

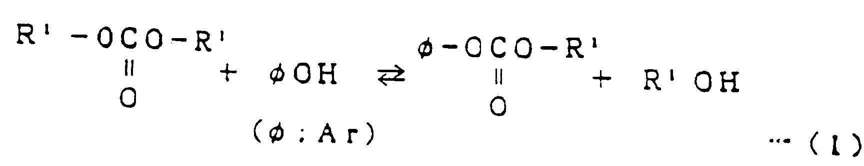

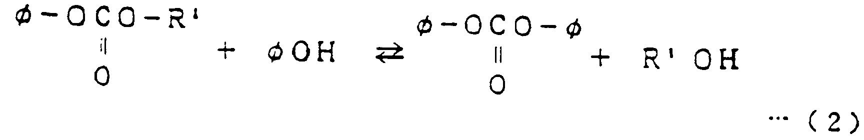

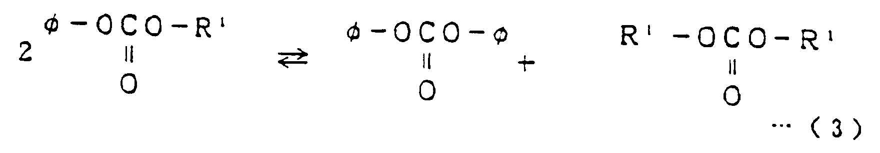

Specific examples of alkyl aryl carbonates that can be obtained by means of reaction (1) include methyl phenyl carbonate, ethyl phenyl carbonate, propyl phenyl carbonate, allyl phenyl carbonate, butyl phenyl carbonate, pentyl phenyl carbonate, hexyl phenyl carbonate, heptyl phenyl carbonate, octyl tolyl carbonate, nonyl (ethylphenyl) carbonate, decyl (butylphenyl) carbonate, methyl tolyl carbonate, ethyl tolyl carbonate, propyl tolyl carbonate, butyl tolyl carbonate, allyl tolyl carbonate, ethyl xylyl carbonate, methyl (trimethylphenyl) carbonate, methyl (chlorophenyl) carbonate, methyl (nitrophenyl) carbonate, methyl (methoxyphenyl) carbonate, methyl cumyl carbonate, methyl (naphthyl) carbonate, methyl (pyridyl) carbonate, ethyl cumyl carbonate, methyl (benzoylphenyl) carbonate, ethyl xylyl carbonate, benzyl xylyl carbonate, methyl (hydroxyphenyl) carbonate, ethyl (hydroxyphenyl) carbonate, methoxycarbonyloxybiphenyl, methyl (hydroxybiphenyl) carbonate, methyl 2-(hydroxyphenyl)propylphenyl carbonate, and ethyl 2-(hydroxyphenyl)propylphenyl carbonate.

The above-described reaction of dialkyl carbonate and an aromatic hydroxy compound in the presence of a catalyst is conducted in a reactor.

Reactors such as reaction-type distilling columns and continuous reactors equipped with distilling columns can be used, but it is preferred to use a reaction-type distilling column.

It is preferred to use a reaction-type distilling column with a large vapor-liquid boundary to enhance the productivity of the above-mentioned reaction. Specifically, a multistage distilling column having at least two distilling stages can be used, examples including conventional multistage distilling columns such as plate columns, packed columns, and columns combining plates and packing. It is preferred to have the catalyst present in all stages of such multistage distilling columns. When a solid catalyst is used in a packed column, it can comprise part or all of the packing.

The reaction can be conducted with a solvent copresent as desired. Solvents that are inert in the reaction can be used, examples including ethers, alicyclic hydrocarbons, and halogenated aromatic hydrocarbons.

The reaction can also be conducted with inert gases such as nitrogen, helium, and argon copresent.

While the reaction conditions differ according to the type and construction of the reactor and the reaction materials, the reaction is usually conducted at a temperature (internal column temperature) of 50 to 350°C, 100 to 280°C being preferred and 150 to 280°C being especially preferred. The reaction may be conducted at reduced, ordinary, or elevated pressure, but it is usually conducted at pressures ranging from 2,600 Pa to 5.4 MPa. The mean residence time in the reactor is usually 0.001 to 50 h, with a time ranging from 0.01 to 10 h being preferred and 0.05 to 5 h being more preferred.

In the present invention, a single reactor may be used to carry out reactions (1) to (3) for manufacturing aromatic carbonates, or the process may be carried out in two or more reactors. However, it is preferred to use two reactors and to conduct reaction (1), which produces chiefly alkyl aryl carbonate, in the first reactor and reactions (2) and (3), which produce diaryl carbonate, in the second reactor.

Aromatic carbonates produced in a reactor, examples of which are cited hereinabove, are continuously removed from the reactor, usually in a liquid state from the bottom of the reactor. Aromatic carbonates removed from the bottom of the reactor are then sent to a refinement column for refinement

Meanwhile, alcoholic by-products are usually removed from the top of the reactor.

The unreacted raw materials, dialkyl carbonate and aromatic hydroxy compound, are continuously removed from the reactor, separated, recovered, and recycled to the reaction system. This dialkyl carbonate includes dialkyl carbonate that is a by-product of the reaction as well as the original unreacted raw material. Hereinbelow, dialkyl carbonate [by-product] recovered from the reactor may occasionally be referred to as an unreacted raw material.

When unreacted raw materials are recovered and recycled to the reaction system in this manner in the present invention, the unreacted raw materials are separated from alkyl aromatic ether by-products formed in the reactor (mainly in the first reactor when two reactors are used) and then recycled to the reaction system.

This alkyl aromatic ether is a by-product of the reaction of dialkyl carbonate and the aromatic hydroxy compound (Ar¹OH) and is produced at a low rate of selectivity. It can be described by *Ar*¹O*R*¹ or *Ar*¹O*R*². Anisole, such as that shown hereinbelow, is usually the by-product of the reaction of dimethyl carbonate and phenol.

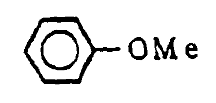

The above-described reaction product (aromatic carbonate), alcoholic by-products, unreacted raw materials (dialkyl carbonate and aromatic hydroxy compound), and alkyl aromatic ether by-products are removed as reaction mixture from the top and bottom of the reactor. And the present invention has a refinement and recycling process in which the various components are separated from the reaction mixture and the unreacted raw materials thus obtained are recycled to the reaction system. Specifically, at least one of the following refinement and recycling processes is carried out
[I] a refinement and recycling process in which alcohols and alkyl aromatic ethers are separated and removed from the reaction mixture removed from the top of the reactor, this reaction mixture containing alcoholic by-products, dialkyl carbonates, and alkyl aromatic ether by-products formed in the reactor, and the dialkyl carbonate thus obtained is recycled to the reaction system and
[II] a refinement and recycling process in which the reaction mixture removed from the bottom of the reactor, this reaction mixture containing aromatic carbonates, dialkyl carbonates, aromatic hydroxy compound, and alkyl aromatic ether by-products formed in the reactor, is distilled, aromatic carbonate is removed from the bottom of the distilling column, alkyl aromatic ethers are separated and removed from the reaction mixture obtained at the top of the distilling column, and the dialkyl carbonate and/or aromatic hydroxy compound thus obtained are returned to the reaction system.
It is possible to use either refinement and recycling process [I] or [II] alone, but it is preferred to use both refinement and recycling processes [I] and [II] together.

Aromatic carbonates, alcohols, dialkyl carbonate, aromatic hydroxy compounds, and alkyl aromatic ethers can usually be separated by distillation. All of the components in the reaction mixture removed from the reactor may be separated in a single distillation apparatus or they may be removed sequentially in two or more distillation apparatuses. For example, when alcohols and alkyl aromatic ethers are separated and removed from the reaction mixture coming off the top of the reactor, the alcohols can be removed in one step, the alkyl aromatic ethers can be removed from this partially refined mixture in the next step, and then the dialkyl carbonate thus obtained can be recycled to the reaction system.

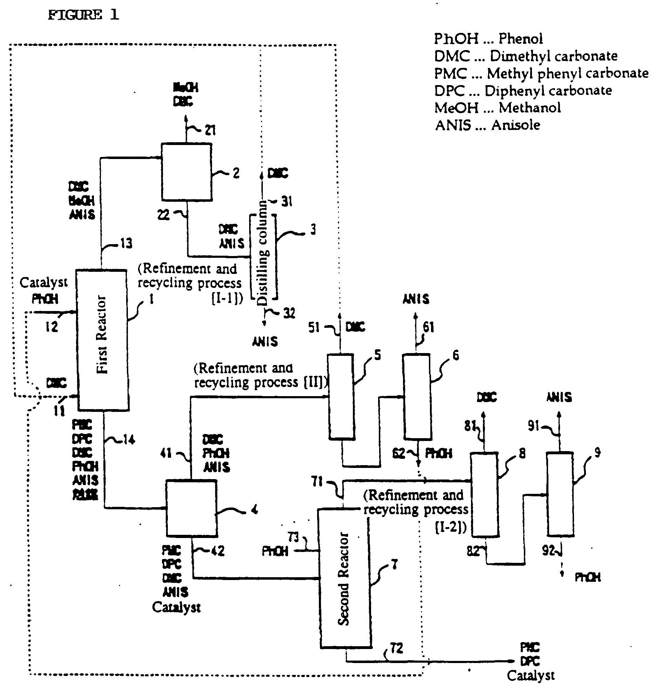

A specific example of the continuous manufacturing method for aromatic carbonates in which two reactors are used to manufacture an aromatic carbonate from dimethvl carbonate and phenol is described hereinbelow with reference to Figure 1.

In Figure 1, dimethyl carbonate is continuously fed to reactor 1 from lower line 11, and phenol and a homogeneous catalyst are continuously fed to the reactor from line 12, as the dimethyl carbonate and phenol are reacted in reactor 1. It should be noted that the raw materials may be introduced through the same line.

The reaction mixture (reaction mixture A hereinbelow) removed from the top 13 of reactor 1 (preferably the top of a column), contains unreacted and newly produced dimethyl carbonate, methyl alcohol by-product and anisole.

This reaction mixture A is led to distilling column 2, where the methyl alcohol by-product is removed via line 21. A portion of the dimethyl carbonate is also removed in this distillation by boiling the methyl alcohol and dimethyl carbonate (7:3) [as an azeotropic mixture].

Components of the distillation residue such as dimethyl carbonate and anisole (an alkyl aromatic ether) are concentrated, and it is therefore possible to remove the anisole with another distillation and obtain highly pure dimethyl carbonate (process [I-1] of the first refinement and recycling process).

Specifically, the distillation residue is fed from the bottom of distilling column 2, through line 22, to distilling column 3, where the dimethyl carbonate and anisole are separated. Anisole is removed via line 32, and the highly pure dimethyl carbonate removed via line 31 can be recycled to reactor 1.

Substances such as dimethyl carbonate (boiling point 90-91°C) and anisole (155.5°C) can be separated easily in refinement and recycling process [I-1] because of the large disparity in their boiling points. Dimethyl carbonate refined in refinement and recycling process [I-1] can be returned to the reaction system for reuse.

Meanwhile, the reaction mixture (reaction mixture B) removed from the bottom of reactor 1, preferably the base of a column, via line 14 usually contains methyl phenyl carbonate, diphenyl carbonate, dimethyl carbonate, phenol, anisole by-product and catalyst.

This reaction fluid is distilled in distilling tower 4, and the distillate, which usually contains dimethyl carbonate, phenol, and anisole, is removed from the top of distilling tower 4 via line 41 leading to refinement and recycling process [II]. It should be noted that the bottom product from the distillation conducted in distilling column 4 contains a small amount of anisole.

In refinement and recycling process [II], dimethyl carbonate, phenol, and anisole are separated by distillation from the distillate obtained from the top of distilling column 4. More specifically, dimethyl carbonate is first distilled off the top of distilling column 5 and can then be recycled via line 51 to reactor 1. Meanwhile, the bottom product removed from the bottom of distilling tower 5 is fed to distilling column 6, where anisole and phenol are separated by distillation. The anisole is distilled off of the top of distilling column 6, and phenol is removed from the bottom and can be returned to reactor 1 via line 62.

Meanwhile, the reaction fluid removed from the bottom of distilling tower4 via line 42, which contains substances such as dimethyl carbonate, phenol, and anisole as well as methyl phenyl carbonate and diphenyl carbonate, is led to the second reactor 7.

Diphenyl carbonate is produced in the second reactor 7 by reacting chiefly methyl phenyl carbonate and phenol (the phenol is fed via line 73). A methyl phenyl carbonate reaction for producing diphenyl carbonate also occurs in this second reactor 7.

Anisole, as well as dimethyl carbonate and phenol, is distilled from the top 71 of the second reactor 7. This distillate is led to refinement and recycling process [I-2], and the anisole is removed in the same manner as in refinement and recycling process [II], enabling highly pure dimethyl carbonate and phenol to be recovered. Specifically, distillate from reactor top 71 is [again] distilled in distilling column 8, enabling dimethyl carbonate from distilling column top 81 to be recycled to line 11 of reactor 1. In addition, phenol and anisole are led from distilling column bottom 82 to distilling column 9, where they are separated by distillation, thus allowing the separated phenol to be recycled from the distilling column bottom 92 to line 12 of reactor 1. Anisole is removed from distilling column top 91.

Meanwhile, aromatic carbonate obtained in reactor 7 can be refined in a refinement column (not shown).

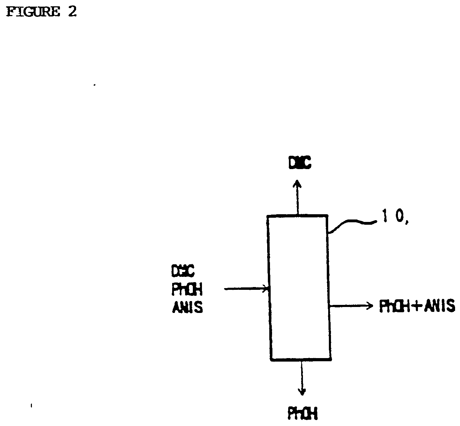

In the above-described refinement and recycling processes [II] and [I-2], anisole may be separated and removed by separating the reaction mixture into dimethyl carbonate, phenol, and a mixture of anisole and phenol, as shown in Figure 2, using, for example, a multistage column 10.

In the method for continuously manufacturing aromatic carbonates of the present invention, aromatic carbonates can be manufactured continuously and efficiently by removing specific by-products (alkyl aromatic ethers) from recovered unreacted raw materials and then returning the raw materials to the reaction system.

A concrete working example of the present invention is described hereinbelow, but the invention is not limited by these examples.

### Example 1

Aromatic carbonate was manufactured by a process using two reactors configured as in Figure 1.

A 500 mL autoclave having a tray-type distilling column, 3 m high and 2 inches in diameter, with 40 sieve trays was used as the device (reactor) for producing methyl phenyl carbonate (PMC).

Reaction fluid was continuously removed from a pipe provided for that purpose at the base of the autoclave's column. The low-boiling-point component containing alcoholic by-products was removed from the top of the distilling column and liquefied in a condenser. A portion of the low-boiling-point component was removed, and the balance was refluxed through the distilling column (reflux ratio = 1). The autoclave and distilling column were heated with electric ovens, controlled in such a way that the temperature at the base of the distilling column was 206°C. Heaters were used to heat the raw material feed lines.

Initially, phenol (PhOH) and, as catalyst, Ti(OPh)₄ were continuously fed to the twentieth stage of the distilling column in amounts of 293 g/h (280 g of phenol and 13 g of catalyst), and the portion of the column above the twentieth stage was used for distillation.

Dimethyl carbonate (DMC) was continuously fed to the autoclave in amounts of 1,220 g/h. A continuous operation was conducted in such a way that the DMC/PhOH molar ratio at the base of the column was approximately 1 without distilling phenol off the top of the column. As a result, product was obtained at a rate of 973 g/h from the top of the distilling column and 540 g/h from the base of the column. Ninety-five grams per hour of methyl phenyl carbonate and approximately 0.3 g/h of anisole were produced in the liquor at the base of the column.

DMC and methanol (MeOH) were the main components, and approximately 0.1 g/h of anisole was present at the top of the column early in the reaction.

Distillate was distilled from the top of the distilling column at ordinary pressure, the azeotropic mixture of MeOH and DMC was separated, and the DMC component was further refined by distillation and recycled to the device for producing methyl phenyl carbonate.

Meanwhile, the liquor at the base of the column in the device for producing methyl phenyl carbonate was concentrated at a rate of approximately 700 g/h by simple distillation. Since DMC was the main constituent in the low-boiling-point component (approximately 270 g/h), the low-boiling-point component was returned as recovered DMC to the device for producing methyl phenyl carbonate, and the concentrated bottom liquor was fed to the device for producing diphenyl carbonate.

A 500 mL autoclave having a packed distilling column (internal diameter: 2 inches) with 25 theoretical plates was used as the reactor in the process for producing diphenyl carbonate (DPC). The concentrate was fed to the middle of the distilling column, and the reaction was conducted at a column top pressure of 110 torr, column base temperature of 200°C, residence time of 1.5 h, and reflux ratio of 1. About 500 g/h of distillate was obtained from the top of the column, and about 200 g/h of bottom product was obtained from the base of the column.

Since phenol was the main component of the distillate from the top of the column, the distillate was returned as recycled phenol to the methyl phenyl carbonate production process.

As a result, anisole accumulated at a slow rate, and the anisole concentration at the base of the column of the device for producing methyl phenyl carbonate stabilized at 0.5% or less (Table 1).

**Table 1**

| Reaction Time | PMC Concentration (%) | Anisole Concentration (%) |
|---|---|---|
| Early stage of reaction | 17.6 | 0.06 |
| 1 week | 17.4 | 0.5 |
| 2 week | 17.4 | 0.5 |

### Comparative Example 1

In Example 1, distillate from the top of the PMC reaction column was distilled at ordinary pressure, the azeotropic mixture of MeOH and DMC was separated, and the residue was returned without further treatment as DMC to the device for producing methyl phenyl carbonate.

When a continuous operation was conducted under these conditions, the methyl phenyl carbonate concentration declined over time with the accumulation of anisole, as can be seen in Table 2.

**Table 2**

| Reaction Time | PMC Concentration (%) | Anisole Concentration (%) |
|---|---|---|
| Early stage of reaction | 17.6 | 0.06 |
| 1 week | 17.2 | 2 |
| 2 week | 16.5 | 5 |

### Brief Description of the Drawings

Figure 1 A process flow chart showing an example of a mode of carrying out the continuous manufacturing method for aromatic carbonates of the invention.

Figure 2 An alternative example of the distillation process in refinement recycling process [II] or [1-2].

## Claims

1. A continuous manufacturing method for aromatic carbonates **characterized by** carrying out at least one of the refinement and recycling processes hereinbelow in a method for continuously manufacturing aromatic carbonates in a reactor by reacting dialkyl carbonate described by general formula (i) 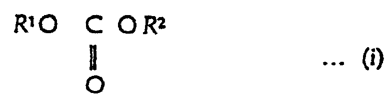 where R¹ and R² are alkyl groups, alkenyl groups, alicyclic groups, alkyl groups containing alicyclic groups, or aralkylgroups, R¹ and R² may be the same or different, and they may form a ring, or be substituted with cyano groups and halogen and an aromatic hydroxy compound in the presence of a catalyst,
while continuously removing aromatic carbonate reaction product, alcoholic by-products, dialkyl carbonate, and aromatic hydroxy compound and while recovering and recycling to the reaction system dialkyl carbonate and aromatic hydroxy compound:
[I] a refinement and recycling process in which alcohols and alkyl aromatic ethers are separated arid removed from the reaction mixture removed from the top of the reactor, this reaction mixture containing alcoholic by-products, dialkyl carbonates, and alkyl aromatic ether by-products formed in the reactor, and the dialkyl carbonate thus obtained is recycled to the reaction system
and
[II] a refinement and recycling process in which the reaction mixture removed from the bottom of the reactor, this reaction mixture containing aromatic carbonates, dialkyl carbonates, aromatic hydroxy compound, and alkyl aromatic ether by-products formed in the reactor, is distilled, aromatic carbonate is removed from the bottom of the distilling column, alkyl aromatic ethers are separated and removed from the reaction mixture obtained at the top of the distilling column, and the dialkyl carbonate and/or aromatic hydroxy compound thus obtained are returned to the reaction system.

2. The method in Claim 1, **characterized by** the fact that the aromatic carbonates are alkyl aryl carbonates, diaryl carbonates, or mixtures of them.

3. The method in Claim 1, **characterized by** the fact that the dialkyl carbonate is dimethyl carbonate or diethyl carbonate.

4. The method in Claim 1, **characterized by** the fact that the aromatic hydroxy compound is phenol, *m*- or *p*-cresol.

5. The method in Claim 1, **characterized by** the fact that the alkyl aromatic ethers are anisole, phenetole, methyl tolyl ether, or ethyl tolyl ether.

6. The method in Claim 1, **characterized by** the fact that
two reactors are used and
in the first reactor, alkyl aryl carbonates are produced as the aromatic carbonates by reacting dialkyl carbonate and an aromatic hydroxy compound in the presence of a catalyst
and then in the second reactor, diaryl carbonates are produced as the aromatic carbonate by either reacting the alkyl aryl carbonate obtained in the first reactor or by reacting the alkyl aryl carbonate and an aromatic hydroxy compound.

## Patentansprüche

1. Kontinuierliches Herstellungs-Verfahren für aromatische Carbonate, **gekennzeichnet durch** Ausführen mindestens eines der folgenden Reinigungs- und Rückführungs-Verfahren in einem Verfahren zum kontinuierlichen Herstellen aromatischer Carbonate in einem Reaktor **durch** Umsetzen von Dialkylcarbonat, das **durch** die allgemeinen Formel (i) beschrieben ist, 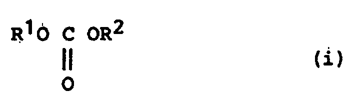 worin R¹ und R² Alkylgruppen, Alkenylgruppen, alicyclische Gruppen, alicyclische Gruppen enthaltende Alkylgruppen oder Aralkylgruppen sind, R¹ und R² gleich oder verschieden sein können und sie einen Ring bilden oder mit Cyangruppen und Halogen substituiert sein können,
und einer aromatischen Hydroxyverbindung in Gegenwart eines Katalysators,
während kontinuierlich aromatisches Carbonat-Reaktionsprodukt, alkoholische Nebenprodukte, Dialkylcarbonat und aromatische Hydroxyverbindung entfernt und während Dialkylcarbonat und aromatische Hydroxyverbindung gewonnen und zu dem Reaktionssystem zurückgeführt werden:
[I] ein Reinigungs- und Rückführungs-verfahren, bei dem Alkohole und aropiatische Alkylether getrennt und aus der Reaktionsmischung entfernt werden, die aus dem Oberteil des Reaktors entfernt wird, wobei diese Reaktionsmischung alkoholische Nebenprodukte, Dialkylcarbonate und aromatische Alkylether-Nebenprodukte enthält, die im Reaktor gebildet wurden, und das so erhaltene Dialkylcarbonat zum Reationssystem zurückgeführt wird, und
[II] ein Reinigungs- und Rückführungs-Verfahren, bei dem die am Boden des Reaktors abgenommene Reaktionsmischung, die im Reaktor gebildete aromatische Carbonate, Dialkylcarbonate, aromatische Hydroxyverbindung und aromatische Alkylether-Nebenprodukte enthält, destilliert wird, aromatisches Carbonat am Boden der Destillationssäule entfernt, aromatische Alkylether abgetrennt und aus der am Oberteil der Destillationssäule erhaltenen Reaktionsmischung entfernt, und das so erhaltene Dialkylcarbonat und/oder die aromatische Hydroxyverbindung zum Reaktionssystem zurückgeführt werden.

2. Verfahren nach Anspruch 1, **gekennzeichnet durch** die Tatsache, dass die aromatischen Carbonate Alkylarylcarbonate, Diarylcarbonate oder Mischungen davon sind.

3. Verfahren nach Anspruch 1, **gekennzeichnet durch** die Tatsache, dass das Dialkylcarbonat Dimethylcarbonat oder Diethylcarbonat ist.

4. Verfahren nach Anspruch 1, **gekennzeichnet durch** die Tatsache, dass die aromatische Hydroxyverbindung Phenol, m- oder p-Cresol ist.

5. Verfahren nach Anspruch 1, **gekennzeichnet durch** die Tatsache, dass die aromatischen Alkylether Anisol, Phenetol, Methyltolylether oder Ethyltolylether sind.

6. Verfahren nach Anspruch 1, **gekennzeichnet durch** die Tatsache, dass zwei Reaktoren benutzt werden und im ersten Reator Alkylarylcarbonate als aromatische Carbonate **durch** Umsetzen von Dialkylcarbonat und einer aromatischen Hydroxyverbindung in Gegenwart eines Katalysators erzeugt werden, und dann im zweiten Reaktor Diarylcarbonate als aromatisches Carbonat **durch** Umsetzen entweder des im ersten Reaktor erhaltenen Alkylarylcarbonats oder **durch** Umsetzen des Alkylarylcarbonats und einer aromatischen Hydroxyverbindung hergestellt werden.

## Revendications

1. Procédé de préparation en continu de carbonates aromatiques, **caractérisé en ce que**, dans un procédé de préparation en continu de carbonates aromatiques où l'on fait réagir dans un réacteur un carbonate de dialkyle de formule générale (i) : 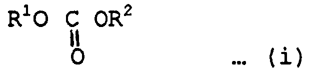 dans laquelle R¹ et R² qui peuvent être identiques ou différents, représentent chacun un groupe alkyle, alcényle, alicyclique, cycloalkylalkyle ou aralkyle, qui peut être substitué par un groupe cyano ou un atome d'halogène, R¹ et R² pouvant en outre former un cycle, avec un composé aromatique hydroxylé, en présence d'un catalyseur, tout en enlevant de manière continuelle le carbonate aromatique produit de la réaction, des sous-produits alcooliques, du carbonate de dialkyle et du composé aromatique hydroxylé et en récupérant et recyclant dans le système réactionnel le carbonate de dialkyle et le composé aromatique hydroxylé, on réalise au moins l'un des procédés de purification et de recyclage indiqués ci-après :
[I] un procédé de purification et de recyclage dans lequel les alcools et les éthers alkyliques aromatiques sont séparés et éliminés du mélange réactionnel ôté par le sommet du réacteur, ce mélange réactionnel contenant des sous-produits alcooliques, du carbonate de dialkyle et des éthers alkyliques aromatiques formés comme sous-produits dans le réacteur, et le carbonate de dialkyle ainsi obtenu est recyclé dans le système réactionnel, et
[II] un procédé de purification et de recyclage dans lequel le mélange réactionnel ôté par le bas du réacteur, qui contient des carbonates aromatiques, du carbonate de dialkyle, du composé aromatique hydroxylé et des éthers alkyliques aromatiques formés comme sous-produits dans le réacteur, est distillé, le carbonate aromatique est ôté par le bas de la colonne de distillation, les éthers alkyliques aromatiques sont séparés et ôtés du mélange réactionnel obtenu au sommet de la colonne de distillation, et le carbonate de dialkyle et/ou le composé aromatique hydroxylé ainsi obtenus sont renvoyés au système réactionnel.

2. Procédé selon la revendication 1, **caractérisé en ce que** les carbonates aromatiques sont des carbonates d'alkyle et d'aryle, des carbonates de diaryle ou des mélanges de ces produits.

3. Procédé selon la revendication 1, **caractérisé en ce que** le carbonate de dialkyle est le carbonate de diméthyle ou le carbonate de diéthyle.

4. Procédé selon la revendication 1, **caractérisé en ce que** le composé aromatique hydroxylé est le phénol, le m-crésol ou le p-crésol.

5. Procédé selon la revendication 1, **caractérisé en ce que** les éthers alkyliques aromatiques sont l'anisole, le phénétole, l'oxyde de méthyle et de tolyle ou l'oxyde d'éthyle et de tolyle.

6. Procédé selon la revendication 1, **caractérisé en ce que** l'on utilise deux réacteurs et l'on produit dans le premier réacteur des carbonates d'aryle et d'alkyle en tant que carbonates aromatiques, en faisant réagir un carbonate de dialkyle et un composé aromatique hydroxylé en présence d'un catalyseur, et on produit ensuite dans le second réacteur des carbonates de diaryle en tant que carbonates aromatiques, en faisant réagir le carbonate d'aryle et d'alkyle obtenu dans le premier réacteur ou en faisant réagir le carbonate d'aryle et d'alkyle et un composé aromatique hydroxylé.
